(19) Europäisches Patentamt
European Patent Office
Office européen des brevets

(11) EP 4 458 768 A1

(12) # EUROPEAN PATENT APPLICATION
published in accordance with Art. 153(4) EPC

(43) Date of publication:
**06.11.2024 Bulletin 2024/45**

(21) Application number: **22915822.5**

(22) Date of filing: **20.12.2022**

(51) International Patent Classification (IPC):
***C01B 33/18*** (2006.01) ***A61K 8/25*** (2006.01)
***A61K 8/73*** (2006.01) ***A61K 8/893*** (2006.01)
***A61K 8/894*** (2006.01) ***A61Q 1/00*** (2006.01)
***A61Q 3/02*** (2006.01) ***A61Q 5/00*** (2006.01)
***C01B 33/40*** (2006.01) ***C01B 33/42*** (2006.01)

(52) Cooperative Patent Classification (CPC):
**A61K 8/00; A61K 8/02; A61K 8/25; A61K 8/73; A61K 8/893; A61K 8/894; A61Q 1/00; A61Q 3/02; A61Q 5/00; A61Q 19/00; C01B 33/18; C01B 33/40; C01B 33/42**

(86) International application number:
**PCT/JP2022/046869**

(87) International publication number:
**WO 2023/127598 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.12.2021 JP 2021212999**

(71) Applicant: **Kao Corporation**
**Tokyo 103-8210 (JP)**

(72) Inventors:
- **ASANO, Moeko**
  **Wakayama-shi, Wakayama 640-8580 (JP)**
- **TEZUKA, Hikaru**
  **Wakayama-shi, Wakayama 640-8580 (JP)**
- **ISHIYAMA, Shogo**
  **Toyohashi-shi, Aichi 441-8074 (JP)**
- **TAKAI, Masanori**
  **Wakayama-shi, Wakayama 640-8580 (JP)**

(74) Representative: **Hoffmann Eitle**
**Patent- und Rechtsanwälte PartmbB**
**Arabellastraße 30**
**81925 München (DE)**

(54) **SURFACE-COATED PARTICLES**

(57) The present invention relates to a surface-coated particle in which a portion or all of the surface of a particle (A) is coated with a surface treatment agent (B), wherein a dynamic friction coefficient of the surface-coated particle is 0.50 or less, when a 5% by mass ethanol dispersion of the surface-coated particle is applied onto a polyurethane substrate in an amount of 2 $mg/cm^2$, and dried for 24 hours under atmospheric pressure at 23°C, followed by measurement under conditions of a load of 3.53 N, a moving speed of 10 mm/sec, a moving distance of 50 mm, 30 reciprocations, and a temperature of 25±2°C, and the standard deviation of the dynamic friction coefficient is 0.020 or less.

EP 4 458 768 A1

## Description

### Technical Field

The present invention relates to surface-coated particles, a surface modifier, a cosmetic product, a surface modification method, a method for producing the surface-coated particles, and a method for producing texture-improving particles.

### Background Art

Cosmetic products such as cosmetics, skin care products, toiletry products, and perfumes are sometimes blended with synthetic polymer fine particles from the viewpoint of improving the texture.

Here, the synthetic polymer fine particles are difficult to decompose in the natural environment and may adversely affect the ecosystem, so that texture-improving particles that can be an alternative to synthetic polymer fine particles are needed.

Examples of such texture-improving particles blended with cosmetic products include inorganic particles, the surface of which is coated with a silicone compound. Examples of techniques relating to such surface-coated particles include those described in JP 09-301826 A (Patent Literature 1) and International Publication No. WO2020/230650 (Patent Literature 2).

Patent Literature 1 describes inorganic particles, the surface of which is coated with a poly(N-acyl alkylene imine)-modified silicone.

Patent Literature 2 describes a composite surface-treated inorganic powder in which a base powder mainly composed of an inorganic material has been surface-treated with a cationic surfactant and an amino-modified silicone.

### Summary of Invention

The present invention relates to the following [1] to [6].

[1] A surface-coated particle, in which a portion or all of the surface of a particle (A) is coated with a surface treatment agent (B), wherein

a dynamic friction coefficient of the surface-coated particle is 0.50 or less, when a 5% by mass ethanol dispersion of the surface-coated particle is applied onto a polyurethane substrate in an amount of 2 mg/cm$^2$, and dried for 24 hours under atmospheric pressure at 23°C, followed by measurement under conditions of a load of 3.53 N, a moving speed of 10 mm/sec, a moving distance of 50 mm, 30 reciprocations, and a temperature of 25$\pm$2°C, and a standard deviation of the dynamic friction coefficient is 0.020 or less.

[2] A surface modifier, comprising the surface-coated particle according to [1] above.
[3] A cosmetic product, comprising the surface-coated particle according to [1] above.
[4] A surface modification method, comprising a step of applying the surface-coated particle according to [1] above, the surface modifier according to [2] above, or the cosmetic product according to [3] above to a surface of object.
[5] A method for producing the surface-coated particle according to [1] above, comprising a step of mixing the particle (A) with a surface treatment agent solution comprising the surface treatment agent (B) and a solvent.
[6] A method for producing a texture-improving particle, comprising a step of selecting, as a texture-improving particle, a particle having a dynamic friction coefficient of 0.50 or less as measured by the foregoing method and a standard deviation of the dynamic friction coefficient of 0.020 or less, from among the surface-coated particles in which a portion or all of the surface of a particle (A) is coated with a surface treatment agent (B).

### Description of Embodiments

There is a demand for texture-improving particles having a good texture and being capable of reducing the amount of synthetic polymer fine particles used.

One embodiment of the present invention relates to providing surface-coated particles, a surface modifier, and a cosmetic product having a good texture and being capable of reducing the amount of synthetic polymer fine particles used, and a surface modification method using the surface-coated particles, the surface modifier, or the cosmetic product.

Further, one embodiment of the present invention relates to providing a method for producing surface-coated particles having a good texture and being capable of reducing the amount of synthetic polymer fine particles used.

Furthermore, one embodiment of the present invention relates to providing a method for producing texture-improving particles having a good texture and being capable of reducing the amount of synthetic polymer fine particles used.

Accordingly, one embodiment of the present invention can provide the surface-coated particles, a surface modifier, and a cosmetic product having a good texture and being capable of reducing the amount of synthetic polymer fine particles used, and a surface modification method using the surface-coated particles, the surface modifier, or the cosmetic product.

Further, one embodiment of the present invention can provide a method for producing surface-coated particles having a good texture and being capable of reducing the amount of synthetic polymer fine particles used.

Furthermore, one embodiment of the present invention can provide a method for producing texture-improving particles having a good texture and being capable of reducing the amount of synthetic polymer fine particles used.

[Surface-coated particles]

The surface-coated particles of the present invention are surface-coated particles in which a portion or all of the surface of the particles (A) is coated with the surface treatment agent (B), wherein a dynamic friction coefficient of the surface-coated particles is 0.50 or less, when a 5% by mass ethanol dispersion of the surface-coated particles is applied onto a polyurethane substrate in an amount of 2 $mg/cm^2$, and dried for 24 hours under atmospheric pressure at 23°C, followed by measurement under conditions of a load of 3.53 N, a moving speed of 10 mm/sec, a moving distance of 50 mm, 30 reciprocations, and a temperature of 25±2°C, and the standard deviation of the dynamic friction coefficient is 0.020 or less. Here, in the measurement of the dynamic friction coefficient of the surface-coated particles and the dynamic friction coefficient, for example, a 3 cm×3 cm indenter to which a polyurethane substrate has been fixed can be used as a measuring jig.

The surface-coated particles of the present invention have a good texture. Therefore, the surface-coated particles of the present invention can improve the texture of a cosmetic product by blending them in the cosmetic product or using them as the cosmetic product. Furthermore, the use of the surface-coated particles of the present invention as a surface modifier can modify the surface of skin, hair, nails, etc., to have a good texture.

In addition, the surface-coated particles of the present invention are environmentally friendly and the amount of synthetic polymer fine particles used can be reduced, because particles can be used as core particles that do not correspond to synthetic polymer fine particles.

As used herein, the expression "a portion or all of the surface of the particles (A) is coated with the surface treatment agent (B)" means a state such that the surface treatment agent (B) is adsorbed to at least a portion of the surface of the particles (A) via intermolecular force or ionic bond. The term "adsorption" as used herein does not include a mode in which the particles (A) and the surface treatment agent (B) are covalently bonded.

Whether or not the surface treatment agent (B) is adsorbed to at least a portion of the surface of the particles (A) is determined by a known method. For example, when the amount of the surface treatment agent (B) adsorbed to 100 parts by mass of the particles (A), which is calculated by "measurement of the amount of the surface treatment agent adsorbed to the inorganic particles" described in Examples, is greater than 0 part by mass, preferably 0.1 parts by mass or more, and more preferably 0.5 parts by mass or more, it is determined that the surface treatment agent (B) is adsorbed to the surface of the inorganic particles (A).

As used herein, the term "texture" refers to the smoothness and no creakiness of particles when the particles are applied to skin, hair, nails, etc. The smoother and less creakiness, the better the texture. The term "smoothness" refers to a smooth, non-powdery texture, and "creakiness" refers to a feel of catching when a finger touches and moves over the skin, hair, nails, etc.

The dynamic friction coefficient of the surface-coated particles of the present invention is 0.50 or less, and is preferably 0.45 or less, more preferably 0.43 or less, further preferably 0.40 or less, further preferably 0.35 or less, further preferably 0.32 or less, further preferably 0.30 or less, further preferably 0.29 or less, further preferably 0.28 or less, and further preferably 0.26 or less from the viewpoint of further improving the texture.

Although the lowest limit of the dynamic friction coefficient is not particularly limited, and may be, for example, 0.10 or more, may be 0.13 or more, may be 0.16 or more, and may be 0.18 or more.

Specifically, the dynamic friction coefficient of the surface-coated particles of the present invention can be measured by a method described in Examples.

The standard deviation of the dynamic friction coefficient of the surface-coated particles of the present invention is 0.020 or less, and, is preferably 0.018 or less, more preferably 0.017 or less, and further preferably 0.016 or less from the viewpoint of further improving the texture.

The lower limit of the standard deviation of the dynamic friction coefficient is not particularly limited, and may be, for example, 0.001 or more, may be 0.003 or more, may be 0.005 or more, or may be 0.006 or more.

Specifically, the standard deviation of the dynamic friction coefficient of the surface-coated particles of the present invention can be measured by a method described in Examples.

The content of a synthetic polymer that is solid at 25°C and water-insoluble in the surface-coated particles of the present invention is preferably less than 1% by mass, more preferably less than 0.5% by mass, further preferably less than 0.1% by mass, and further preferably less than 0.01% by mass, and the surface-coated particles of the present invention are preferably substantially free of the synthetic polymer from the viewpoint of further improving the environmental compatibility. As used herein, the expression "substantially free of the synthetic polymer" means that the synthetic polymer is not intentionally added, but this does not exclude the presence of a small amount of the synthetic polymer as an impurity.

As used herein, the term "water-insoluble synthetic polymer" refers to a synthetic polymer the dissolution amount of which is 2 g or less when 10 g of the synthetic polymer is mixed with 1,000 mL of water with pH 7 at 20°C followed by 24 hours of stirring, in accordance with the OECD guideline 120.

The term "solid at 25°C" refers to a state of not having fluidity in a bulk state under an environment of 1 atm and 25°C.

The volume median particle size ($D_{50}$) of the surface-coated particles of the present invention is preferably more than 1 μm, more preferably more than 2 μm, and further preferably more than 3 μm, and, preferably 30 μm or less, more preferably 20 μm or less, further preferably 15 μm or less, and further preferably 12 μm or less from the viewpoint of further improving the texture.

Here, the volume median particle size ($D_{50}$) described in the present invention is a 50% median diameter measured by a laser diffraction/scattering method using a particle size distribution analyzer, and can specifically be measured by a method described in Examples.

In the surface-coated particles of the present invention, the surface treatment agent (B) is preferably adsorbed to the surface of the particles (A) via intermolecular force or ionic bond, from the viewpoint of further improving the texture.

In the present specification, the presence or absence of adsorption due to intermolecular force or ionic bond between the particles (A) and the surface treatment agent (B) can be confirmed by a known method, for example, based on a change in the amount adsorbed after washing of the surface-coated particles with a solvent in which the surface treatment agent (B) is soluble, and can be confirmed specifically by a method described in Examples.

Each component constituting the surface-coated particles of the present invention and the method for producing the surface-coated particles will be sequentially described below.

<Particles (A)>

The particles (A) preferably comprise at least one selected from the group consisting of inorganic particles (A1) and polysaccharide particles (A2) from the viewpoint of further improving the texture and environmental compatibility of the surface-coated particles. Since the inorganic particles (A1) and the polysaccharide particles (A2) do not correspond to synthetic polymer fine particles, the surface-coated particles comprising at least one selected from the group consisting of the inorganic particles (A1) and the polysaccharide particles (A2) as core particles have improved environmental compatibility and is more suitable as an alternative material to synthetic polymer fine particles.

The total content of the inorganic particles (A1) and the polysaccharide particles (A2) in the particles (A) is, from the viewpoint of further improving the texture and environmental compatibility of the surface-coated particles, preferably 60% by mass or more, more preferably 70% by mass or more, further preferably 80% by mass or more, further preferably 90% by mass or more, further preferably 95% by mass or more, and further preferably 98% by mass or more, and, is preferably 100% by mass or less.

Further, the content of the synthetic polymer fine particles in the particles (A) is, from the viewpoint of further improving the texture and environmental compatibility of the surface-coated particles, preferably 40% by mass or less, more preferably 30% by mass or less, further preferably, 20% by mass or less, further preferably 10% by mass or less, further preferably 5% by mass or less, and further preferably 2% by mass or less, and further preferably the particles (A) are substantially free of synthetic polymer fine particles. Here, the expression "substantially free of synthetic polymer fine particles" means that the particles (A) can comprise synthetic polymer fine particles that are unintentionally contained as impurities.

The inorganic particles (A1) preferably comprise silica particles or silicate mineral particles from the viewpoint of further improving the texture of the surface-coated particles, more preferably comprise at least one of particles selected from the group consisting of silica particles, talc particles and mica particles, further preferably comprise at least one of particles selected from the group consisting of silica particles and mica particles, and, from the viewpoint of further suppressing the stickiness upon drying, further preferably comprise silica particles, and further preferably comprise porous silica particles.

The silicate mineral particles preferably comprise at least one selected from the group consisting of talc particles, mica particles, sericite particles, smectite particles, montmorillonite particles, bentonite particles and kaolin particles, more preferably comprise at least one selected from the group consisting of talc particles and mica particles, and further preferably comprise mica particles, from the viewpoint of further improving the texture of the surface-coated particles.

The polysaccharide particles (A2) preferably comprise cellulose particles.

When the inorganic particles (A1) comprise porous silica particles, the specific surface area of the porous silica particles is preferably 5 $m^2/g$ or more, and more preferably 10 $m^2/g$ or more, and, preferably 1,000 $m^2/g$ or less, and more preferably 900 $m^2/g$ or less from the viewpoint of suppressing the disintegration of the porous silica particles and further improving the texture of the surface-coated particles. The specific surface area is a BET specific surface area measured according to JIS Z 8830:2013.

When the inorganic particles (A1) comprise porous silica particles, the pore volume of the porous silica particles is preferably 0.1 mL/g or more, more preferably 0.3 mL/g or more, and further preferably 0.5 mL/g or more, and, preferably 5 mL/g or less, more preferably 3 mL/g or less, and further preferably 2 mL/g or less from the viewpoint of suppressing the disintegration of the porous silica particles and further improving the texture of the surface-coated particles. In addition, from the viewpoint of no creakiness, it is preferably 0.3 mL/g or more, more preferably 0.5 mL/g or more, further preferably 0.8 mL/g or more, and further preferably 0.9 mL/g or more, and, preferably 5 mL/g or less, more preferably 3 mL/g or less, further preferably 2 mL/g or less, and further preferably 1.5 mL/g or less.

The pore volume can be measured by a gas adsorption method. Examples of the gas adsorption method include JIS Z 8831-2: 2010 (Pore size distribution and porosity of solid materials - Part 2: Analysis of mesopores and macropores by gas adsorption), and JIS Z 8831 -3: 2010 (Pore size distribution and porosity of solid materials - Part 3: Analysis of micropores by gas adsorption).

The content of at least one of particles selected from the group consisting of the inorganic particles (A1) and the polysaccharide particles (A2) in the particles (A) is preferably 50% by mass or more, more preferably 60% by mass or more, further preferably 70% by mass or more, further preferably 80% by mass or more, further preferably 90% by mass or more, further preferably 95% by mass or more, and preferably 100% by mass or less, from the viewpoint of further improving the texture of the surface-coated particles.

The content of at least one of particles selected from the group consisting of silica particles and silicate mineral particles in the inorganic particles (A1) is preferably 50% by mass or more, more preferably 60% by mass or more, further preferably 70% by mass or more, further preferably 80% by mass or more, further preferably 90% by mass or more, further preferably 95% by mass or more, and preferably 100% by mass or less, from the viewpoint of further improving the texture of the surface-coated particles.

The content of cellulose particles in the polysaccharide particles (A2) is preferably 50% by mass or more, more preferably 60% by mass or more, further preferably 70% by mass or more, further preferably 80% by mass or more, further preferably 90% by mass or more, further preferably 95% by mass or more, and preferably 100% by mass or less from the viewpoint of further improving the texture of the surface-coated particles.

Examples of the shape of the particles (A) include spherical, plate-like, flaky, and irregular shapes. Among these, from the viewpoint of further improving the texture of the surface-coated particles, the particles (A) are preferably spherical, plate-like or flaky particles, and more preferably spherical or flaky particles.

More specifically, when the particles (A) are silica particles or polysaccharide particles (A2), they are more preferably spherical, and when the particles (A) are silicate mineral particles, they are more preferably flaky.

The volume median particle size ($D_{50}$) of the particles (A) is preferably 1 μm or more, more preferably 2 μm or more, and further preferably 3 μm or more, and, preferably 30 μm or less, more preferably 20 μm or less, further preferably 15 μm or less, and further preferably 12 μm or less, from the viewpoint of further improving the texture of the surface-coated particles.

<Surface treatment agent (B)>

The surface treatment agent (B) comprises preferably at least one selected from the group consisting of a modified silicone (b1) liquid at 25°C (hereinafter also referred to as "modified silicone (b1)") and an water-soluble cationic polymer (b2), from the viewpoint of further improving the texture and environmental compatibility of the surface-coated particles, and comprises more preferably the modified silicone (b1) liquid at 25°C, from the viewpoint of further suppressing stickiness upon drying.

As used herein, the term "liquid at 25°C" refers to a state of having fluidity in a bulk state under an environment of 1 atm and 25°C.

Since the modified silicone (b1) liquid at 25°C and the water-soluble cationic polymer (b2) do not correspond to "synthetic polymer that is solid at 25°C and water-insoluble", the surface-coated particles comprising at least one selected from the group consisting of the modified silicone (b1) liquid at 25°C and the water-soluble cationic polymer (b2) as the surface treatment agent (B) have improved environmental compatibility and are more suitable as an alternative material to synthetic polymer fine particles.

The surface treatment agent (B) comprises preferably 50% by mass or more, more preferably 70% by mass or more, further preferably 80% by mass or more, further preferably 90% by mass or more, further preferably 95% by mass or more, and preferably 100% by mass or less of at least one selected from the group consisting of the modified

silicone (b1) and the water-soluble cationic polymer (b2), from the viewpoint of further improving the texture and environmental compatibility of the surface-coated particles.

The modified silicone (b1) liquid at 25°C comprises preferably at least one selected from the group consisting of a polyether-modified silicone, a polyglycerin-modified silicone, a branched polyglycerol-modified silicone, and an alkyl glyceryl ether-modified silicone, from the viewpoint of improving the texture of the surface-coated particles, and comprises more preferably at least one selected from the group consisting of a polyether-modified silicone, a polyglycerin-modified silicone, and a branched polyglycerol-modified silicone.

The HLB (hydrophilic-lipophilic balance) of the modified silicone (b1) is preferably 1.0 or more, more preferably 2.0 or more, further preferably 2.5 or more, and is preferably 20.0 or less, more preferably 18.0 or less, further preferably 16.0 or less, further preferably 15.5 or less, further preferably 15.0 or less, further preferably 10.0 or less, and further preferably 5.0 or less, from the viewpoint of further improving the texture of the surface-coated particles.

Here, the HLB value is a value that represents the affinity of the modified silicone (b1) for water and oil, and can be found from the following formula according to the Griffin method.

HLB = 20 × [(molecular weight of hydrophilic group contained in modified silicone (b1))/(molecular weight of modified silicone (b1))]

Examples of the hydrophilic group include a hydroxy group and an ethyleneoxy group.

The viscosity of the modified silicone (b1) at 25°C is preferably 50 mPa·s or more, more preferably 100 mPa·s or more, further preferably 200 mPa·s or more, and further preferably 400 mPa·s or more, and, preferably 500,000 mPa·s or less, more preferably 100,000 mPa·s or less, and further preferably 80,000 mPa·s or less.

The above viscosity can be measured at 25°C using a Brookfield viscometer based on JIS Z8803:2011 "Methods for viscosity measurement of liquid".

The polyether-modified silicone has a structure in which the side chains and/or terminal hydrocarbon groups of silicone oil are substituted with polyether groups.

The polyether group of the polyether-modified silicone is preferably a polyethyleneoxy group, a polypropyleneoxy group, or a polyalkyleneoxy group formed of an ethyleneoxy group (EO) and a propyleneoxy group (trimethyleneoxy group or propane-1,2-diyloxy group; PO) added in a block form or randomly, and is more preferably a polyethyleneoxy group. As the polyether-modified silicone, a compound formed of a polyether group grafted to the silicone main chain, a compound formed of a silicone and a polyether group bonded in a block form, or the like can be used.

The viscosity of the polyether-modified silicone at 25°C is preferably 50 mPa·s or more, more preferably 100 mPa·s or more, further preferably 200 mPa·s or more, further preferably 400 mPa or more, and further preferably 450 mPa·s or more, and, preferably 3,000 mPa.s or less, more preferably 2,000 mPa.s or less, further preferably 1,000 mPa.s or less, further preferably 750 mPa·s or less, and further preferably 600 mPa·s or less, from the viewpoint of further improving the texture of the surface-coated particles.

The above viscosity can be measured at 25°C using a Brookfield viscometer based on JIS Z8803:2011 "Methods for viscosity measurement of liquid".

Specific examples of the polyether-modified silicone include PEG-3 dimethicone, PEG-9 dimethicone, PEG-9 methyl ether dimethicone, PEG-10 dimethicone, PEG-11 methyl ether dimethicone, PEG-12 dimethicone, PEG/PPG-20/22 butyl ether dimethicone, PEG-32 methyl ether dimethicone, PEG-9 polydimethylsiloxyethyl dimethicone, lauryl PEG-9 polydimethylsiloxyethyl dimethicone, cetyl PEG/PPG-10/1 dimethicone, PEG/PPG-30/10 dimethicone, and a linear polyether-modified silicone in which dimethyl silicone units and polyether units are alternately copolymerized.

Examples of a commercially available polyether-modified silicone include KF series manufactured by Shin-Etsu Chemical Co., Ltd. (for example, KF-6004, KF-6011, KF-6012, KF-6013, KF-6015, KF-6016, KF-6017, KF-6028, KF-6038, KF-6043, and KF-6048), and DOWSII, series manufactured by Dow Chemical Japan Ltd. (BY25-339, SH3775M, and FZ-2203).

The polyglycerin-modified silicone refers to a silicone having a monovalent polyglyceryl group in its structure, and examples thereof include polyglyceryl-3 disiloxane dimethicone, polyglyceryl-3 polydimethylsiloxyethyl dimethicone, and lauryl polyglyceryl-3 polydimethylsiloxyethyl dimethicone. Here, in the present specification, a branched polyglycerol-modified silicone is excluded from examples of the polyglycerin-modified silicone.

Examples of a commercially available polyglycerin-modified silicone include KF series manufactured by Shin-Etsu Chemical Co., Ltd. (for example, KF-6100, KF-6104, KF-6106, and KF-6105).

The viscosity of the polyglycerin-modified silicone at 25°C is preferably 500 mPa·s or more, more preferably 1,000 mPa·s or more, further preferably 2,000 mPa·s or more, and further preferably 3,000 mPa·s or more, and, preferably 20,000 mPa·s or less, more preferably 10,000 mPa·s or less, further preferably 5,000 mPa·s or less, and further preferably 4,000 mPa·s or less, from the viewpoint of further improving the texture of the surface-coated particles.

The above viscosity can be measured at 25°C using a Brookfield viscometer based on JIS Z8803:2011 "Methods

for viscosity measurement of liquid".

An example of the branched polyglycerol-modified silicone is a compound represented by the following formula (1) having a structure in which branched polyglycerol chains are bonded to both ends of dimethylpolysiloxane via linking groups each containing an oxyphenylene group.

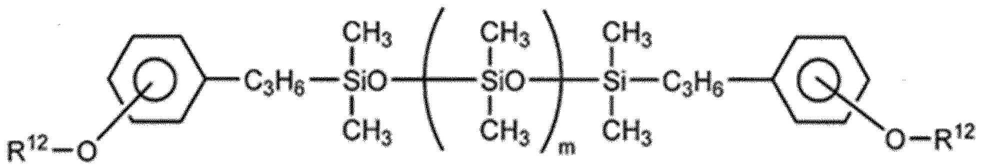 (1)

In formula (1), each $R^{12}$ independently represents a branched polyglycerol chain, and "m" represents an integer of 0 or more and 10,000 or less.

In formula (1), the binding mode of an oxygen atom and a trimethylene group ($-C_3H_6-$) in each phenylene group portion is not particularly limited, and may be ortho-, meta- or para-position. Further, "m" represents an integer of 0 or more and 10,000 or less, and preferably 1 or more and 300 or less. $R^{12}$ represents a branched polyglycerol chain, is composed of glycerol units represented by the following structural formulas (2) to (5), and has at least one glycerol unit having a branched structure represented by structural formula (2), and the end is a glycerol unit represented by structural formula (5). The average total number of bonds of glycerol units in the branched polyglycerol chain is 3 or more and 200 or less, and preferably 3 or more and 30 or less.

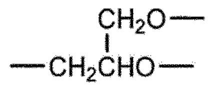 (2)

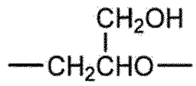 (3)

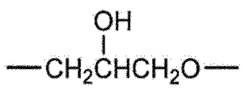 (4)

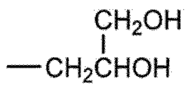 (5)

The viscosity of the branched polyglycerol-modified silicone at 25°C is preferably 10,000 mPa·s or more, more preferably 20,000 mPa·s or more, further preferably 40,000 mPa·s or more, further preferably 60,000 mPa·s or more, and further preferably 70,000 mPa·s or more, and, preferably 500,000 mPa·s or less, more preferably 100,000 mPa·s or less, and further preferably 80,000 mPa.s or less, from the viewpoint of further improving the texture of the surface-coated particles. The above viscosity can be measured at 25°C using a Brookfield viscometer based on JIS Z8803:2011 "Methods for viscosity measurement of liquid".

The branched polyglycerol-modified silicone represented by formula (1) can be produced by the method described in JP 2004-339244 A, which involves adding 2,3-epoxy-1-propanol to a modified silicone having phenyl groups substituted with hydroxy groups at both ends in the presence of an acidic or basic catalyst to perform graft polymerization for production, or other generally known methods.

Examples of a commercially available branched polyglycerol-modified silicone include SOFCARE GS-G (manufactured by Kao Corporation).

Examples of the alkyl glyceryl ether-modified silicone include the one represented by the following formula (6).

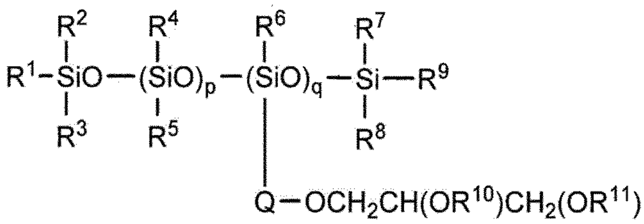

(6)

In formula (6), "Q" represents a divalent hydrocarbon group having 3 or more and 20 or less carbon atoms, $R^1$ to $R^9$ may be the same or different, and each represent a hydrogen atom, a linear or branched hydrocarbon group having 1 or more and 32 or less carbon atoms, or a phenyl group, $R^{10}$ and $R^{11}$ may be the same or different, and each represent a hydrogen atom or a linear or branched hydrocarbon group having 1 or more and 32 or less carbon atoms, multiple $R^{10}$ and $R^{11}$ in the same molecule may be different from each other. Further, "p" represents a number of 1 or more and 500 or less, and "q" represents a number of 1 or more and 50 or less. In addition, "p" and "q" represent preferably numbers such that the content of the alkyl glyceryl ether group [$-Q-OCH_2-CH(OR^{10})-CH_2(OR^{11})$] in the molecule is 1% by mass or more and 50% by mass or less.

In formula (6), the divalent hydrocarbon group represented by "Q" having 3 or more and 20 or less carbon atoms is preferably a linear or branched alkylene group having 3 or more and 20 or less carbon atoms. More specific examples thereof include: linear alkylene groups such as trimethylene, tetramethylene, pentamethylene, hexamethylene, heptamethylene, octamethylene, nonamethylene, decamethylene, undecamethylene, dodecamethylene, tetradecamethylene, hexadecamethylene and octadecamethylene; and branched alkylene groups such as propylene, 2-methyltrimethylene, 2-methyltetramethylene, 2-methylpentamethylene and 3-methylpentamethylene.

Examples of the linear or branched hydrocarbon group having 1 or more and 32 or less carbon atoms in the definitions of $R^1$ to $R^{11}$ include: linear alkyl groups such as methyl, ethyl, propyl, butyl, pentyl, hexyl, octyl, decyl, undecyl, dodecyl, tetradecyl, hexadecyl, octadecyl, eicosyl, doeicosyl, tetraeicosyl, hexaeicosyl, octaeicosyl, and triacontyl; and branched alkyl groups such as isopropyl, sec-butyl, tert-butyl, neopentyl, 1-ethylpropyl, and 1-heptyldecyl. In the present specification, $R^1$ to $R^9$ are preferably linear or branched alkyl groups each having 1 or more and 25 or less carbon atoms (some of which may be hydrogen atoms), and are more preferably linear or branched alkyl groups each having 1 or more and 22 or less carbon atoms (some of which may be hydrogen atoms). In addition, $R^{10}$ and $R^{11}$ are preferably hydrogen atoms or alkyl groups each having 1 or more and 5 or less carbon atoms, and further preferably hydrogen atoms.

Furthermore, "p" and "q" are preferably numbers such that the content of the alkyl glyceryl ether group [$-Q-OCH_2-CH(OR^{10})-CH_2(OR^{11})$] is 1% by mass or more and 50% by mass or less, more preferably numbers such that the content is 5% by mass or more and 40% by mass or less, and further preferably numbers such that the content is 10% by mass or more and 30% by mass or less. Specifically, "p" is in the range of 1 or more and 500 or less, preferably in the range of 10 or more and 30 or less, and "q" is in the range of 1 or more and 50 or less, and preferably in the range of 1 or more and 30 or less in terms of the availability of the raw material organopolysiloxane, the operability during production, etc.

The viscosity of the alkyl glyceryl ether-modified silicone at 25°C is preferably 1,000 mPa.s or more, more preferably 2,000 mPa.s or more, further preferably 3,000 mPa.s or more, further preferably 4,000 mPa·s or more, further preferably 5,000 mPa·s or more, and further preferably 6,000 mPa·s or more, and, preferably 20,000 mPa·s or less, more preferably 10,000 mPa·s or less, further preferably 8,000 mPa·s or less, and further preferably 7,000 mPa·s or less from the viewpoint of further improving the texture of the surface-coated particles. The above viscosity can be measured at 25°C using a Brookfield viscometer based on JIS Z8803:2011 "Methods for viscosity measurement of liquid".

Such an alkyl glyceryl ether-modified silicone can be produced according to the methods described in JP H4-134013 A and JP 2005-194523 A that involve reacting organohydrogenpolysiloxane having at least one silicon-hydrogen bond with a corresponding alkenyl glyceryl ether.

Examples of a commercially available alkyl glyceryl ether-modified silicone include SI-UGE and SOFCARE RS-U (manufactured by Kao Corporation).

These modified silicones (b 1) may be used alone or in combination of two or more.

The water-soluble cationic polymer (b2) preferably comprises at least one selected from the group consisting of a cationic polymer having a cellulose backbone, a cationic vinyl-based polymer, chitin compounds/chitosan compounds, and halogenated hexadimethrine, more preferably comprises at least one selected from the group consisting of a cationic polymer having a cellulose backbone, a cationic vinyl-based polymer, chitin compounds/chitosan compounds, hexadimethrine bromide and hexadimethrine chloride, more preferably comprises at least one selected from the group consisting of a cationic polymer having a cellulose backbone, a cationic vinyl-based polymer and chitin compounds/chitosan compounds, and further preferably comprises at least one selected from the group consisting of a cationic polymer having a cellulose backbone and a cationic vinyl-based polymer, from the viewpoint of further improving

the texture and environmental compatibility of the surface-coated particles.

As used herein, the term "water-soluble polymer" refers to a polymer, the dissolution amount of which is more than 2 g when 10 g of the polymer is mixed with 1,000 mL of water with pH 7 at 20°C and then the mixture is left to stand for 24 hours.

The water-soluble cationic polymer (b2) preferably has a basic group such as primary to tertiary amino groups, a quaternary ammonium group, and a hydrazino group, more preferably a tertiary amino group, or a quaternary ammonium group. As the basic group, those neutralized with acids such as hydrochloric acid, sulfuric acid, nitric acid, acetic acid, formic acid, maleic acid, fumaric acid, citric acid, tartaric acid, adipic acid and lactic acid are included.

In the present specification, water-soluble cationic silicones such as water-soluble amino-modified silicones are excluded from examples of the water-soluble cationic polymer (b2).

The weight-average molecular weight Mw of the water-soluble cationic polymer (b2) is preferably 3,000 or more, more preferably 5,000 or more, further preferably 15,000 or more, further preferably 50,000 or more, and further preferably 100,000 or more from the viewpoint of improving the texture of the surface-coated inorganic particles. The Mw is preferably 5 million or less, more preferably 3 million or less, and further preferably 2 million or less.

The weight average molecular weight Mw can be measured by a gel permeation chromatography (GPC) method using polyethylene glycol as a standard substance. For example, the weight-average molecular weight Mw of the cationized hydroxyethyl cellulose, which is the water-soluble cationic polymer (b2), is determined by performing GPC measurement under the following conditions.

<GPC measurement conditions>

Sample concentration: 1 mg/mL
Column: TSKgel $\alpha$-M $\times$ 2 (Tosoh Corporation)
Eluent: 0.15 mol/L aqueous solution of sodium sulfate (containing 1% acetic acid)
Flow rate: 1.0 mL/min
Column temperature: 40°C
Detector: differential refractometer

As used herein, the term "cationic polymer having a cellulose backbone" refers to a polymer that has cationic groups and a cellulose backbone, and is a cation positively charged as a whole. In addition, the cationic group refers to a cationic group or a group that can be ionized to become a cationic group, and examples thereof include a primary amino group, a secondary amino group, a tertiary amino group, and a quaternary ammonium group.

Examples of the cationic polymer having a cellulose backbone include cationized cellulose and cationized cellulose derivatives, and specific examples thereof include cationized cellulose, cationized hydroxyethyl cellulose, cationized hydroxypropyl cellulose, and cationized carboxymethyl cellulose.

The cationic polymer having a cellulose backbone is preferably cationized hydroxyethyl cellulose, and more preferably a polymeric quaternary ammonium salt (INCI name: Polyquaternium-10) obtained by addition of glycidyltrimethylammonium chloride with hydroxyethyl cellulose.

Examples of the cationic vinyl-based polymer include vinylpyrrolidone/dimethylaminoethyl methacrylate copolymer diethyl sulfate, vinylpyrrolidone/dimethylaminopropyl methacrylamide/lauryldimethylaminopropyl methacrylamide copolymer, vinylpyrrolidone/N,N-dimethylaminoethyl methacrylate/alkyl acrylate/tripropylene glycol diacrylate copolymer, polydimethylmethylenepiperidinium chloride, dimethyldiallylammonium chloride/acrylamide copolymer, trimethylammoniopropylacrylamide chloride/dimethylacrylamide copolymer, alkylacrylamide/acrylate/alkylaminoalkylacrylamide/polyethylene glycol methacrylate copolymer, t-butylacrylamide/dimethylacrylamide/dimethylaminopropylacrylamide/methoxypolyethylene glycol methacrylate copolymer, vinylpyrrolidone/N,N-dimethylaminoethyl methacrylate copolymer diethyl sulfate, vinylpyrrolidone/dimethylaminoethyl methacrylate copolymer, N,N-dimethylaminoethyl methacrylate diethyl sulfate/N,N-dimethylacrylamide/polyethylene glycol dimethacrylate copolymer, ammonium-modified hydroxyethyl cellulose, and acrylamide/DMAPA acrylate/methoxy PEG methacrylate copolymer. Among these, a tertiary amino group-containing (meth)acrylic polymer is preferable, and an acrylamide/DMAPA acrylate/methoxy PEG methacrylate copolymer is far preferable.

Examples of chitin compounds/chitosan compounds include chitin/chitosan derivatives such as hydroxypropylchitosan, carboxymethylchitin and carboxymethylchitosan, and chitin and chitosan, with chitosan being preferred.

These water-soluble cationic polymers (b2) may be used alone or in combination of two or more.

The surface treatment agent (B) may comprise a surface treatment agent other than the modified silicone (b1) and the water-soluble cationic polymer (b2). Examples of the surface treatment agent other than the modified silicone (b 1) and the water-soluble cationic polymer (b2) include surface treatment agents applied to cosmetic particles.

The content of at least one selected from the group consisting of the modified silicone (b 1) liquid at 25°C and the water-soluble cationic polymer (b2) in the surface treatment agent (B) is, from the viewpoint of further improving the texture of the surface-coated particles, and, from the viewpoint of further improving the environmental compatibility, preferably 50% by mass or more, more preferably 60% by mass or more, further preferably 70% by mass or more, further preferably 80% by mass or more, further preferably 90% by mass or more, and further preferably 95% by mass or more, and preferably 100% by mass or less.

The content of the synthetic polymer that is solid at 25°C and water-insoluble in the surface treatment agent (B) is preferably less than 10% by mass, more preferably less than 5% by mass, further preferably less than 1% by mass, further preferably less than 0.1% by mass, and further preferably less than 0.01% by mass, and the surface treatment agent (B) is preferably substantially free of the synthetic polymer from the viewpoint of further improving the environmental compatibility.

In the surface-coated particles of the present invention, the amount of the surface treatment agent (B) adsorbed to the particles (A) is preferably 0.1 parts by mass or more, more preferably 0.5 parts by mass or more, further preferably 0.7 parts by mass or more, further preferably 0.9 parts by mass or more, further preferably 1.0 part by mass or more, further preferably 1.5 parts by mass or more, further preferably 2.0 parts by mass or more, further preferably 2.5 parts by mass or more, further preferably 3.5 parts by mass or more, further preferably 4.5 parts by mass or more, further preferably 5.5 parts by mass or more, and further preferably 6.5 parts by mass or more, and, preferably 50 parts by mass or less, more preferably 30 parts by mass or less, further preferably 20 parts by mass or less, further preferably 15 parts by mass or less, further preferably is 13 parts by mass or less, further preferably 10 parts by mass or less, further preferably 8.5 parts by mass or less, and further preferably 7.5 parts by mass or less with respect to 100 parts by mass of the particles (A) from the viewpoint of further improving the texture.

The amount of the surface treatment agent (B) adsorbed can be measured by a method described in Examples.

[Method for producing surface-coated particles]

The surface-coated particles of the present invention can be obtained by coating the particles (A) with the surface treatment agent (B).

Specifically, the method for producing surface-coated particles preferably comprises, for example, a step of mixing particles (A) with a surface treatment agent solution comprising the surface treatment agent (B) and an organic solvent from the viewpoint of coating the particles (A) with the surface treatment agent (B), and more preferably comprises a step of mixing a particle dispersion liquid comprising the particles (A) and solvent A with a surface treatment agent solution comprising the surface treatment agent (B) and solvent B.

Water or an organic solvent can be used as the solvent. Although the organic solvent is not particularly limited, alcohols such as methanol, ethanol, 1-propanol and 2-propanol can be used as an organic solvent. Among the alcohols, at least one selected from the group consisting of ethanol and 2-propanol is preferable, and ethanol is further preferable.

The above solvent A and solvent B may be the same or different from each other as long as they have compatibility with each other.

Solvent A to be used in the particle dispersion liquid is preferably at least one selected from the group consisting of water and ethanol from the viewpoint of dispersibility of the particles (A).

When the surface treatment agent (B) comprises the modified silicone (b1), a solvent to be used in the surface treatment agent solution is preferably an organic solvent from the viewpoint of solubility of the modified silicone (b1), and is more preferably ethanol from the viewpoint of compatibility with solvent A.

When the surface treatment agent (B) comprises the water-soluble cationic polymer (b2), a solvent to be used in the surface treatment agent solution is preferably at least one selected from the group consisting of water and ethanol from the viewpoint of solubility of the water-soluble cationic polymer (b2), and is more preferably water. From the viewpoint of improving the solubility of the water-soluble cationic polymer (b2), an organic acid such as lactic acid may be further added to the surface treatment agent solution.

When the particle dispersion liquid is used, the concentration of the particles (A) in the particle dispersion liquid is preferably 0.5% by mass or more, and more preferably 1.0% by mass or more, and, preferably 40% by mass or less, more preferably 30% by mass or less, further preferably 20% by mass or less, and further preferably 10% by mass or less from the viewpoint of efficient adsorption of the surface treatment agent (B) onto the surface of the particles (A).

When the surface treatment agent solution is used, the concentration of the surface treatment agent (B) in the surface treatment agent solution is preferably 0.5% by mass or more, and more preferably 1.0% by mass or more, and, preferably 40% by mass or less, more preferably 30% by mass or less, further preferably 20% by mass or less, and further preferably 10% by mass or less from the viewpoint of efficient adsorption of the surface treatment agent (B) onto the surface of the particles (A).

The temperature, at which the particles (A) or the particle dispersion liquid is mixed with the surface treatment agent solution, is preferably 5°C or higher, and more preferably 10°C or higher from the viewpoint of efficient adsorption

of the surface treatment agent (B) onto the surface of the particles (A), and the temperature is preferably 70°C or lower, and more preferably 50°C or lower from the viewpoint of suppressing volatilization of the solvent.

Further, the mixing time for mixing the particles (A) or the particle dispersion liquid with the surface treatment agent solution is preferably 5 minutes or longer, and more preferably 10 minutes or longer from the viewpoint of sufficient adsorption of the surface treatment agent (B) onto the surface of the particles (A), and the mixing time is preferably 12 hours or shorter, and more preferably 6 hours or shorter from the viewpoint of production efficiency.

In the step of mixing the particles (A) or the particle dispersion liquid with the surface treatment agent solution, a known stirring device can be used.

The mixture obtained by the above steps is separated using a known method such as centrifugation, filtration, decantation, and drying, so that the surface-coated particles can be recovered.

The obtained surface-coated particles may be classified using a sieve or the like in order to adjust the particle size.

[Surface modifier]

The surface-coated particles of the present invention can be applied to a surface modifier.

Specifically, the surface modifier of the present invention comprises the surface-coated particles of the present invention.

As used herein, the term "surface modifier" refers to an agent capable of improving the texture of the surface of skin, hair, nails and the like.

The content of the surface-coated particles of the present invention in the surface modifier of the present invention is not particularly limited because it can be appropriately set according to the type of the surface modifier, but is, for example, 0.1% by mass or more, and preferably 1% by mass or more, and is, for example, 50% by mass or less, preferably 30% by mass or less, and more preferably 10% by mass or less.

The surface modifier of the present invention can adequately comprise optional components that are used according to the type of the surface modifier, as long as they do not impair the effects of the present invention. Examples of optional components other than the surface-coated particles of the present invention include ultraviolet absorbers, oils, surfactants, water-soluble polymers, thickeners, neutralizers, propellants, pH adjusters, fungicides, anti-inflammatory agents, preservatives, coloring agents, chelating agents, whitening agents, blood circulation promoters, cooling agent, antiperspirants, insect repellents, physiologically active ingredients, salts, moisturizers, antioxidants, flavors, and plant extracts.

Examples of the form of the surface modifier of the present invention include liquid, milky lotion, cream, paste, gel, wax, solid, and multi-layered forms. The surface modifier of the present invention can also be applied to sheet agents, spray agents, and mousse agents, for example.

The surface modifier of the present invention can be applied to, for example, skin (including lips), hair, nails, etc., and can be used preferably by applying the surface modifier.

The content of the synthetic polymer fine particles in the surface modifier of the present invention is preferably less than 0.01% by mass, more preferably less than 0.005% by mass, and further preferably less than 0.001% by mass from the viewpoint of further improving the environmental compatibility.

As used herein, the term "synthetic polymer fine particles" refers to fine particles comprising a 1% by mass or more of synthetic polymer that is solid at 25°C and water-insoluble and having a volume median particle size ($D_{50}$) of 0. 1 $\mu$m or more and 5 mm or less.

The method for producing the surface modifier of the present invention is not particularly limited, and the surface modifier can be produced by stirring and mixing each component using a known device, for example.

[Cosmetic product]

The surface-coated particles of the present invention can be blended with or applied to various cosmetic products.

Specifically, the cosmetic product of the present invention comprises the surface-coated particles of the present invention.

The content of the surface-coated particles of the present invention in the cosmetic product of the present invention is not particularly limited because it can be appropriately set according to the type of the cosmetic product, and is, for example, 0.1% by mass or more, and preferably 1% by mass or more, and, for example, 50% by mass or less, and preferably 30% by mass or less.

As used herein, the term "cosmetic product" refers to a product that comes into direct contact with a human body, such as cosmetics, skin care products, toiletry products, and perfumes.

The cosmetic product of the present invention can adequately contain optional components that are used according to the type of the cosmetic product within a range that does not impair the effects of the present invention.

Examples of the optional components other than the surface-coated particles of the present invention include ultraviolet absorbers, oils, surfactants, water-soluble polymers, thickeners, neutralizers, propellants, pH adjusters, fungicides, anti-inflammatory agents, preservatives, coloring agents, chelating agents, whitening agents, blood circulation promoters, cooling agents, antiperspirants, insect repellents, physiologically active ingredients, salts, moisturizers, antioxidants, flavors, and plant extracts.

Examples of the form of the cosmetic product of the present invention include liquid, milky lotion, cream, paste, gel, wax, solid, and multi-layered forms. The cosmetic product of the present invention can also be applied to sheets, sprays, and mousses, for example.

The cosmetic product of the present invention can be applied to, for example, skin (including lips), hair, and nails, and can be used preferably by applying the cosmetic product.

The content of the synthetic polymer fine particles in the cosmetic product of the present invention is preferably less than 0.01% by mass, more preferably less than 0.005% by mass, and further preferably less than 0.001% by mass from the viewpoint of further improving the environmental compatibility.

The method for producing the cosmetic product of the present invention is not particularly limited. For example, the cosmetic product can be produced by stirring and mixing each component using a known device.

[Surface modification method]

The surface modification method of the present invention comprises a step of applying the surface-coated particles of the present invention, the surface modifier of the present invention, or the cosmetic product of the present invention to a surface of object.

Examples of the surface of object include skin, hair, and nails, and the surface of object is preferably skin.

Examples of the method for applying the surface-coated particles, the surface modifier, or the cosmetic product of the present invention to the surface of object include a method of applying, spraying, or letting it to run over the surface of object, and a method of immersing an object in the surface-coated particles, the surface modifier, or the cosmetic product of the present invention.

[Method for producing texture-improving particles]

The method for producing texture-improving particles of the present invention comprises a step of selecting, as texture-improving particles, particles having a dynamic friction coefficient of 0.50 or less as measured by the following method and a standard deviation of the dynamic friction coefficient of 0.020 or less from among surface-coated particles in which a portion or all of the surface of the particles (A) is coated with the surface treatment agent (B).

(Method)

A 5% by mass ethanol dispersion of the surface-coated particles is applied onto a polyurethane substrate in an amount of 2 mg/cm$^2$, and dried for 24 hours under atmospheric pressure at 23°C, followed by measurement under conditions of a load of 3.53 N, a moving speed of 10 mm/sec, a moving distance of 50 mm, 30 reciprocations, and a temperature of 25±2°C.

According to the method for producing texture-improving particles of the present invention, texture-improving particles having a good texture can be stably obtained.

The texture-improving particles obtained by the method for producing texture-improving particles of the present invention have a good texture. Therefore, the texture-improving particles of the present invention can improve the texture of a cosmetic product by blending them in the cosmetic product or using them as the cosmetic product. Furthermore, through the use of the texture-improving particles of the present invention as a surface-modifier, the surface of skin, hair, nails, etc., can be modified to have a good texture.

Further, since the texture-improving particles of the present invention can use particles that do not correspond to synthetic polymer fine particles as core particles, they are environmentally friendly and suitable as an alternative material to synthetic polymer fine particles.

Preferred aspects of the particles (A), the surface treatment agent (B) and the surface-coated particles in the method for producing texture-improving particles of the present invention, and the preferred ranges of the dynamic friction coefficient and the standard deviation of the dynamic friction coefficient are the same as those for the surface-coated particles of the present invention.

Examples

The present invention will be described below with reference to examples, but the present invention is not

limited to the scope of the examples. In Examples and Comparative Examples, various measurements and evaluations were performed by the following methods.

[Measurement and evaluation methods]

(1) Measurement of amount of surface treatment agent adsorbed to particles before surface coating

The amount of a surface treatment agent adsorbed was found by subjecting samples to thermogravimetry·differential thermal analysis (TG-DTA) using a thermal analyzer manufactured by Rigaku Corporation (Thermo plus EVO2 TG-DTA8122).

The measurement conditions were as follows.

Sample preparation: Surface-coated particles obtained in Examples and Comparative Examples and particles before surface coating (approximately 30 mg (precisely weighed)) Temperature-rising rate: 10°C/min
Measurement temperature range: 35°C to 800°C
Measurement atmosphere: Air (180 mL/min)

From the obtained TG-DTA curve, the amount of the surface treatment agent adsorbed to 100 parts by mass of the particles before surface coating was calculated from the following formula. Here, the amount of the surface treatment agent adsorbed to the surface-coated particles was defined as x % by mass (the total amount of the surface-coated particles is assumed to be 100% by mass).

Amount (parts by mass) of surface treatment agent adsorbed to 100 parts by mass of particles before surface coating = $x/(100-x) \times 100$.

x is calculated from the following formula.
$A \times x/100 + A \times (1-x/100) \times y = B$

A: Mass (mg) of measurement sample of surface-coated particles
B: Reduced mass (mg) of surface-coated particles between 100°C and 800°C
y: Mass reduction rate of particles before surface coating between 100°C and 800°C (= reduced mass of particles before surface coating between 100°C and 800°C/mass of measurement sample of particles before surface coating)

(2) Measurement of volume median particle size ($D_{50}$) of particles before surface coating and surface-coated particles

The particle size distribution of the particles before surface coating and the same of the surface-coated particles were each measured using a laser diffraction/scattering particle size distribution analyzer LA-960 manufactured by HORIBA, Ltd. Distilled water was added to measurement cells so that aqueous solutions of the particles before surface coating and the surface-coated particles had the appropriate range of absorbance. Then the volume median particle size ($D_{50}$) of the particles before surface coating and the same of the surface-coated particles were each calculated from the obtained particle size distribution.

(3) Measurement of dynamic friction coefficient

Dynamic friction coefficients were measured by the method standardized in JIS K7125:1999. The dynamic friction coefficient $\mu_k$ is expressed by Formula 1 below wherein F is the friction force and N is the vertical load.

$$F=\mu_k N \qquad \text{(Formula 1)}$$

A surface property tester ("Tribogear TYPE: 14" manufactured by SHINTO Scientific Co., Ltd.) was used for measurement of $\mu_k$. A surface-modified substrate was fixed to the test stand of a tester with a double-sided tape, unprocessed artificial leather (manufactured by Teijin Cordley Limited., polyurethane substrate, product name: Cordley (registered trademark) ST2923NY, 3 cm $\times$ 10 cm) was fixed to a friction element (3 cm $\times$ 3 cm indenter, "ASTM flat indenter that is attachment to the main body of the surface property tester), a weight of 360 g (vertical load of 3.53 N) was placed in the vertical direction of the friction element, and a reciprocating test was performed. In the reciprocating

test, the artificial leather wound around the friction element was brought into contact with the surface-modified substrate and reciprocated (under conditions of moving speed: 10 mm/sec, moving distance: 50 mm, number of reciprocations: 30 times (5 times of reciprocation was regarded as one measurement, and a total of 6 measurements were performed), and temperature 25±2°C). Then the dynamic friction coefficient obtained at the 30th reciprocation was employed. The artificial leather (polyurethane substrate) of a measuring jig was replaced each time one measurement (5 reciprocations) was completed. The artificial leather was wound around the friction element, and the surplus part was bent and fixed.

Moreover, the surface-modified substrate was produced by the following method.

First, 5% by mass of the particles of Examples and Comparative Examples and 95% by mass of ethanol were added to a 9 mL screw cap container and then cleaned in an ultrasonic bath for 2 minutes to obtain a surface modifier.

Next, 2 mg/cm$^2$ of the thus obtained surface modifier was added drop by drop to artificial leather ("Cordley (registered trademark) ST2923NY manufactured by Teijin Cordley Limited.) composed of 45% by mass of polyurethane and 55% by mass of polyethylene terephthalate, and then spread evenly. Then, the resultant was dried at room temperature (23°C) and atmospheric pressure for 24 hours to obtain a surface-modified substrate.

(4) Measurement of standard deviation of dynamic friction coefficient

The standard deviation of $\mu_k$ was measured in the same manner as the measurement of a dynamic friction coefficient. The standard deviation of a dynamic friction coefficient within the area of 41,000 to 45,000 milliseconds after the test initiation (i.e., the area at the 30th reciprocation in the 6th measurement) in the 6$^{th}$ measurement (i.e., 25$^{th}$ to 30$^{th}$ reciprocations) was employed.

(5) Sensory evaluation

(5-1) No creakiness and smoothness

Under an environment of a temperature of 20°C-25°C and humidity of 25% to 40% RH, 0.2 g of particles obtained in Examples and Comparative Examples was applied evenly to the inside of the forearm using a finger, the applied product was rubbed with a finger to evaluate no creakiness and smoothness, severally, and then the results were scored according to the following criteria.

Three expert panelists evaluated each sample shown in the table below by comparing each texture of "no creakiness" and "smoothness" with those of a reference sample (lauryl methacrylate·ethylene glycol dimethacrylate-sodium methacrylate copolymer particles, volume median particle size ($D_{50}$): 2 μm, produced according to Example 1 of JP 2006-8980 A). The samples evaluated to be equivalent to the reference sample, and felt to be very good in terms of "no creakiness" and "smoothness" were scored "5". Those evaluated to be somewhat inferior to the reference sample, but felt to be good in terms of "no creakiness" and "smoothness" were scored "4". Those evaluated to be inferior to the reference sample, but felt to be normal in terms of "no creakiness" and "smoothness" were scored "3". Those evaluated to be inferior to the reference sample and felt to be poor in terms of "no creakiness" and "smoothness" were scored "2". Those evaluated to be extremely inferior to the reference sample, and felt to be very poor in terms of "no creakiness" and "smoothness" were scored "1". The average values of scores scored by panelists were employed. In addition, each panelist scored in increments of 0.5 including the intermediate score.

Here, the lauryl methacrylate·ethylene glycol dimethacrylate-sodium methacrylate copolymer particles correspond to polymer particles generally used as texture-improving particles.

[Production of surface-coated particles]

Example 1

To a 500 mL beaker, 2 g of a surface treatment agent, polyglycerin-modified silicone 1 (polyglyceryl-3 polydimethylsiloxyethyl dimethicone, "KF-6104" manufactured by Shin-Etsu Chemical Co., Ltd., viscosity at 25°C: 3,300 mPa·s) and 98 g of ethanol were added, thereby preparing a polyglycerin-modified silicone solution.

To a separately prepared 500 mL beaker, 2 g of inorganic particles, silica 1 (porous silica particles, "HCS 160M5" manufactured by JGC Catalysts and Chemicals Ltd., volume median particle size ($D_{50}$): 5 μm, pore volume: 0.7 mL/g) and 98 g of ethanol were added, and then treated with an ultrasonic homogenizer (ULTRASONIC HOMOGENIZER US-600E, manufactured by NISSEI Corporation) at 200 W for 5 minutes, thereby obtaining a particle dispersion liquid.

The polyglycerin-modified silicone solution was added to the obtained particle dispersion liquid, and then the mixture was stirred with a magnetic stirrer at room temperature and 600 rpm for 1 hour. The resulting mixture was separated into particles (dispersoid) and a solution (dispersion medium) by centrifuging at 3,000 rpm for 30 minutes

using a centrifuge (HITACHI, CR21G III). After removing the supernatant, the precipitate was dried under reduced pressure at 50°C overnight to obtain surface-coated particles. Each evaluation was performed on the obtained surface-coated particles. The results are shown in the tables.

Examples 2, 5, 6 and 8 and Comparative Example 1

Surface-coated particles were obtained respectively in the same manner as in Example 1, except that the types of inorganic particles and surface treatment agents were changed to those shown in the tables. Each evaluation was performed on the obtained surface-coated particles. The results are shown in the tables below.

Examples 3, 7 and 9

Surface-coated particles were obtained respectively in the same manner as in Example 1, except that the types of inorganic particles and surface treatment agents were changed to those shown in the tables below, and "98 g of ethanol" was changed to "98 g of water". Each evaluation was performed on the obtained surface-coated particles. The results are shown in the tables below.

Example 4

Except that the types of inorganic particles and surface treatment agents were changed to those shown in the tables below, "98 g of ethanol" was changed to "98 g of water", and 40 mg of 90% lactic acid (product name: Musashino Lactic Acid 90, manufactured by Musashino Chemical Laboratory, Ltd.) was added to the obtained cationic vinyl-based polymer 1 solution, surface-coated particles were obtained in the same manner as in Example 1. Each evaluation was performed on the obtained surface-coated particles. The results are shown in the tables below.

Comparative Example 2

Except that "cellulose 1 (cellulose particles, DAITO KASEI KOGYO CO., LTD. "CELLULOBEADS D-5", volume median particle size ($D_{50}$): 5 μm)" was used instead of "silica 1", and ethanol was used instead of "polyglycerin-modified silicone 1", cellulose particles were obtained in the same manner as in Example 1. Each evaluation was performed on the obtained cellulose particles. The results are shown in the tables below.

Table 1

| Example Comparative Example | Particles before surface coating | Surface treatment agent | Physical properties of coating on particles | | Sensory evaluation | |
|---|---|---|---|---|---|---|
| | | | Dynamic friction coefficient (μk) | Standard deviation of μk | Smoothness | No creakiness |
| Example 1 | Silica 1 | Polyglycerin-modified silicone 1 | 0.21 | 0.009 | 4.8 | 4.8 |
| Example 2 | Silica 1 | Branched polyglycerol-modified silicone 1 | 0.25 | 0.015 | 4.8 | 4.8 |
| Example 3 | Silica 1 | Cationized hydroxyethyl cellulose 1 | 0.27 | 0.009 | 4.8 | 4.5 |
| Example 4 | Silica 1 | Cationicvinyl-based polymer 1 | 0.24 | 0.007 | 4.8 | 4.5 |
| Example 5 | Silica 1 | Polyether-modified silicone 1 | 0.25 | 0.016 | 4.8 | 5.0 |
| Example 6 | Mica 1 | Polyether-modified silicone 1 | 0.29 | 0.009 | 4.8 | 4.8 |
| Example 7 | Mica 1 | Cationized hydroxyethyl cellulose 1 | 0.30 | 0.011 | 4.8 | 4.5 |

(continued)

| Example Comparative Example | Particles before surface coating | Surface treatment agent | Physical properties of coating on particles | | Sensory evaluation | |
|---|---|---|---|---|---|---|
| | | | Dynamic friction coefficient (μk) | Standard deviation of μk | Smoothness | No creakiness |
| Example 8 | Talc 1 | Polyether-modified silicone 1 | 0.42 | 0.010 | 4.5 | 4.0 |
| Example 9 | Silica 1 | Hexadimethrine bromide 1 | 0.29 | 0.008 | 3.3 | 3.0 |
| Comparative Example 1 | Titanium oxide 1 | Polyether-modified silicone 1 | 0.58 | 0.012 | 2.0 | 1.0 |
| Comparative Example 2 | Cellulose 1 | | 0.25 | 0.023 | 1.7 | 2.0 |

Table 2

| Example Comparative Example | Surface-coated particles | |
|---|---|---|
| | Adsorption amount of surface treatment agent (part by mass) | Volume median particle size (μm) |
| Example 1 | 12 | 5 |
| Example 2 | 8 | 5 |
| Example 3 | 1 | 5 |
| Example 4 | 1 | 5 |
| Example 5 | 7 | 5 |
| Example 6 | 3 | 10 |
| Example 7 | 6 | 10 |
| Example 8 | 3 | 6 |
| Example 9 | 2 | 5 |
| Comparative Example 1 | 0.4 | 1 |
| Comparative Example 2 | 0 | 5 |

The ingredients listed in the tables are as follows.

(Particles)

- Silica 1 (porous silica particles, "HCS 160M5" manufactured by JGC Catalysts and Chemicals Ltd., volume median particle size ($D_{50}$): 5 μm, pore volume: 0.7 mL/g, spherical)
- Mica 1 (mica particles, "Y-1800" manufactured by Yamaguchi Mica Co., Ltd., volume median particle size ($D_{50}$): 10 μm, flaky)
- Talc 1 (talc particles, "JA-13R" manufactured by Asada Milling Co., Ltd., volume median particle size ($D_{50}$): 6 μm, flaky)
- Titanium oxide 1 (titanium oxide particles, "MP-100" manufactured by Tayca Corporation, volume median particle size ($D_{50}$): 1 μm)
- Cellulose 1 (cellulose particles, "CELLULOBEADS D-5" manufactured by Daito Kasei Kogyo Co., Ltd., volume median particle size ($D_{50}$): 5 μm)

(Surface treatment agent)

- · Polyglycerin-modified silicone 1 (polyglyceryl-3 polydimethylsiloxyethyl dimethicone, "KF-6104" manufactured by Shin-Etsu Chemical Co., Ltd., viscosity at 25°C: 3,300 mPa·s)
- · Branched polyglycerol-modified silicone 1 (bis(polyglyceryl-3 oxyphenylpropyl) dimethicone, "SOFCARE GS-G" manufactured by Kao Corporation, viscosity at 25°C: 77,000 mPa·s)
- · Polyether-modified silicone 1 (PEG-10 dimethicone, "KF-6017", manufactured by Shin-Etsu Chemical Co., Ltd., HLB: 4.5, viscosity at 25°C: 490 mPa·s)
- · Cationized hydroxyethyl cellulose 1 (polyquaternium-10, "POIZ C-150L" manufactured by Kao Corporation)
- · Cationic vinyl-based polymer 1 (acrylamide/DMAPA acrylate/methoxy PEG methacrylate copolymer, produced according to Production Example 1 below)
- · Hexadimethrine bromide ("HCS160M5" manufactured by FUJIFILM Wako Pure Chemical Corporation, Mw: 5,000 to 10,000)

Production example 1

A four-necked flask equipped with a reflux condenser, a liquid feeder, a thermometer, a nitrogen gas inlet tube and a stirrer was heated to 50°C in an oil bath. A monomer solution was obtained by dissolving 100 parts by mass of monomers (mass ratio of monomers: N-tert-butylacrylamide/N,N-dimethylacrylamide/methoxypolyethylene glycol monomethacrylate/N-(3-dimethylaminopropyl)acrylamide = 55/23/20/2) in 75 parts by mass of ethanol to obtain a monomer solution. Further, a 0.2 mol % initiator (V-65B) with respect to the monomers was dissolved in ethanol so that the concentration was 5% by mass to obtain an initiator solution. The resulting monomer solution and the initiator solution were simultaneously added drop by drop to the flask over 130 minutes under a nitrogen atmosphere, maintained at 50°C for 130 minutes, and then further maintained at 80°C for 120 minutes for reaction. After polymerization, the ethanol solution of the polymer was poured into n-hexane for reprecipitation and purification, and then vacuum-dried at 80°C, thereby giving a cationic vinyl-based polymer 1 in a solid state.

(Reagent used)

- · N-tert-butyl acrylamide (manufactured by Shinryo Corporation)
- · N,N-dimethylacrylamide (manufactured by KJ Chemicals Corporation.)
- · Methoxypolyethylene glycol monomethacrylate (average number of added moles of ethylene oxide: 9 (NK Ester M-90G, manufactured by Shin-Nakamura Chemical Co., Ltd.)
- · N-(3-dimethylaminopropyl) acrylamide (manufactured by KJ Chemicals Corporation.)
- · V-65B: 2,2'-azobis-(2,4-dimethylvaleronitrile) (manufactured by FUJIFILM Wako Pure Chemical Corporation.)
- · Ethanol (manufactured by FUJIFILM Wako Pure Chemical Corporation)
- · n-hexane (manufactured by FUJIFILM Wako Pure Chemical Corporation)

The above tables demonstrate that the surface-coated particles of Examples are excellent in smoothness and no creakiness, and have a good texture. In addition, the surface-coated particles of Examples comprise particles that do not correspond to synthetic polymer fine particles as core particles, and the surface treatment agent does not comprise any synthetic polymer that is solid at 25°C and insoluble in water. Therefore, the surface-coated particles are found to be environmentally friendly and suitable as an alternative material to synthetic polymer fine particles.

Industrial Applicability

According to one embodiment of the present invention, surface-coated particles, a surface modifier, and a cosmetic product having a good texture and being capable of reducing the amount of synthetic polymer fine particles used, and a surface modification method using the surface-coated particles, the surface modifier, or the cosmetic product can be provided.

Further, one embodiment of the present invention can provide a method for producing surface-coated particles having a good texture and being capable of reducing the amount of synthetic polymer fine particles used.

Furthermore, one embodiment of the present invention can provide a method for producing texture-improving particles having a good texture and being capable of reducing the amount of synthetic polymer fine particles used.

## Claims

1. A surface-coated particle, in which a portion or all of the surface of a particle (A) is coated with a surface treatment agent (B), wherein

   a dynamic friction coefficient of the surface-coated particle is 0.50 or less, when a 5% by mass ethanol dispersion of the surface-coated particle is applied onto a polyurethane substrate in an amount of 2 $mg/cm^2$, and dried for 24 hours under atmospheric pressure at 23°C, followed by measurement under conditions of a load of 3.53 N, a moving speed of 10 mm/sec, a moving distance of 50 mm, 30 reciprocations, and a temperature of 25±2°C, and a standard deviation of the dynamic friction coefficient is 0.020 or less.

2. The surface-coated particle according to claim 1, wherein the particle (A) comprises at least one selected from the group consisting of an inorganic particle (A1) and a polysaccharide particle (A2).

3. The surface-coated particle according to claim 2, wherein the inorganic particle (A1) comprises a silica particle or a silicate mineral particle.

4. The surface-coated particle according to claim 2 or 3, wherein the polysaccharide particle (A2) comprises a cellulose particle.

5. The surface-coated particle according to any one of claims 1 to 4, wherein the surface treatment agent (B) comprises at least one selected from the group consisting of a modified silicone (b 1) liquid at 25°C and a water-soluble cationic polymer (b2).

6. The surface-coated particle according to claim 5, wherein the surface treatment agent (B) comprises 50% by mass or more of at least one selected from the group consisting of the modified silicone (b 1) and the water-soluble cationic polymer (b2).

7. The surface-coated particle according to claim 5 or 6, wherein the modified silicone (b 1) comprises at least one selected from the group consisting of a polyether-modified silicone, a polyglycerin-modified silicone, a branched polyglycerol-modified silicone, and an alkyl glyceryl ether-modified silicone.

8. The surface-coated particle according to any one of claims 5 to 7, wherein the water-soluble cationic polymer (b2) comprises at least one selected from the group consisting of a cationic polymer having a cellulose backbone, a cationic vinyl-based polymer, and chitin compounds/chitosan compounds.

9. The surface-coated particle according to any one of claims 1 to 8, wherein the amount of the surface treatment agent (B) adsorbed to the particle (A) is 0.1 parts by mass or more and 50 parts by mass or less with respect to 100 parts by mass of the particle (A).

10. A surface modifier, comprising the surface-coated particle according to any one of claims 1 to 9.

11. A cosmetic product, comprising the surface-coated particle according to any one of claims 1 to 9.

12. A surface modification method, comprising a step of applying the surface-coated particle according to any one of claims 1 to 9, the surface modifier according to claim 10, or the cosmetic product according to claim 11 to a surface of object.

13. The surface modification method according to claim 12, wherein the surface of object is skin.

14. A method for producing the surface-coated particle according to any one of claims 1 to 9, comprising a step of mixing the particle (A) with a surface treatment agent solution comprising the surface treatment agent (B) and a solvent.

15. A method for producing a texture-improving particle, comprising a step of selecting, as a texture-improving particle, a particle having a dynamic friction coefficient of 0.50 or less as measured by the following method and a standard deviation of the dynamic friction coefficient of 0.020 or less, from among the surface-coated particles in which a portion or all of the surface of a particle (A) is coated with a surface treatment agent (B),
(Method)

a 5% by mass ethanol dispersion of the surface-coated particle is applied onto a polyurethane substrate in an amount of 2 $mg/cm^2$, and dried for 24 hours under atmospheric pressure at 23°C, followed by measurement under conditions of a load of 3.53 N, a moving speed of 10 mm/sec, a moving distance of 50 mm, 30 reciprocations, and a temperature of 25±2°C.

**INTERNATIONAL SEARCH REPORT**

International application No.
**PCT/JP2022/046869**

**A. CLASSIFICATION OF SUBJECT MATTER**

***C01B 33/18***(2006.01)i; ***A61K 8/25***(2006.01)i; ***A61K 8/73***(2006.01)i; ***A61K 8/893***(2006.01)i; ***A61K 8/894***(2006.01)i; ***A61Q 1/00***(2006.01)i; ***A61Q 3/02***(2006.01)i; ***A61Q 5/00***(2006.01)i; ***C01B 33/40***(2006.01)i; ***C01B 33/42***(2006.01)i
FI: C01B33/18 C; A61K8/25; A61K8/73; A61K8/893; A61K8/894; A61Q1/00; A61Q3/02; A61Q5/00; C01B33/40; C01B33/42

According to International Patent Classification (IPC) or to both national classification and IPC

**B. FIELDS SEARCHED**

Minimum documentation searched (classification system followed by classification symbols)

C01B33/12-33/46; A61K8/00-8/99; A61Q1/00-90/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580/JSTChina (JDreamIII)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2019-119720 A (MIYOSHI KASEI, INC.) 22 July 2019 (2019-07-22) claims 1-9, paragraphs [0014], [0019]-[0026], examples | 1-3, 5-6, 9-14<br>1-3, 5-6, 9-15 |
| X<br>Y | JP 2006-36981 A (TOPY IND., LTD.) 09 February 2006 (2006-02-09) claims 1-7, paragraphs [0037], [0038], examples | 1-3, 9-14<br>1-3, 9-15 |
| X<br>Y | JP 2004-155978 A (SHIN ETSU CHEM. CO., LTD.) 03 June 2004 (2004-06-03) claims 1-8, paragraphs [0012], [0013], [0020], examples | 1-7, 9-14<br>1-7, 9-15 |
| X<br>Y | JP 2004-169015 A (SHIN ETSU CHEM. CO., LTD.) 17 June 2004 (2004-06-17) claims 1-7, paragraphs [0034], [0035], [0038], examples | 1-7, 9-14<br>1-7, 9-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 March 2023** | **20 March 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/046869**

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2005-289971 A (L'OREAL SA) 20 October 2005 (2005-10-20)<br>paragraphs [0015], [0024], [0025] | 1-6, 8, 10-14<br>1-6, 8-15 |
| X<br>Y | JP 2013-53088 A (POLA CHEMICAL INDUSTRIES, INC.) 21 March 2013 (2013-03-21)<br>claims 1-3, paragraph [0009], examples | 1-3, 5-6, 9-14<br>1-6, 9-15 |
| Y | WO 2021/210472 A1 (AGC SI-TECH CO., LTD.) 21 October 2021 (2021-10-21)<br>paragraphs [0007], [0030], [0031] | 1-15 |
| A | KR 10-2020-0049137 A (EASTHILL CO., LTD.) 08 May 2020 (2020-05-08) | 1-15 |

Form PCT/ISA/210 (second sheet) (January 2015)

**INTERNATIONAL SEARCH REPORT**
**Information on patent family members**

International application No.
**PCT/JP2022/046869**

| Patent document cited in search report | Publication date (day/month/year) | Patent family member(s) | Publication date (day/month/year) |
|---|---|---|---|
| JP 2019-119720 A | 22 July 2019 | (Family: none) | |
| JP 2006-36981 A | 09 February 2006 | (Family: none) | |
| JP 2004-155978 A | 03 June 2004 | EP 1424373 A2<br>claims 1-8, paragraphs [0020], [0021], [0028], examples<br>US 2004/0091440 A1 | |
| JP 2004-169015 A | 17 June 2004 | EP 1416016 A1<br>claims 1-7, paragraphs [0033], [0034], [0037], examples<br>KR 10-2004-0038865 A<br>US 2004/0091439 A1 | |
| JP 2005-289971 A | 20 October 2005 | US 2005/0129640 A1<br>paragraphs [0033], [0067]-[0089]<br>BR PI0405183 A<br>CA 2485331 A1<br>CN 1669550 A<br>EP 1532967 A1<br>FR 2862222 A1<br>MX PA04011443 A | |
| JP 2013-53088 A | 21 March 2013 | (Family: none) | |
| WO 2021/210472 A1 | 21 October 2021 | (Family: none) | |
| KR 10-2020-0049137 A | 08 May 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 9301826 A
- WO 2020230650 A
- JP 2004339244 A
- JP H4134013 A
- JP 2005194523 A
- JP 2006008980 A